# FASCICULE DE BREVET EUROPEEN

(11) **EP 2 300 403 B1**
(45) Date de publication et mention de la délivrance du brevet: **23.01.2013**
(21) Numéro de dépôt: 09749770.5
(22) Date de dépôt: 15.05.2009
(51) Int. Cl.: C07C 37/82, C07C 41/36, C07C 43/23

(54) **PROCEDE DE SEPARATION DE COMPOSES PHENOLIQUES SOUS FORME SALIFIEE**
VERFAHREN ZUR AUFTRENNUNG PHENOLISCHER VERBINDUNGEN IN SALZFORM
METHOD OF SEPARATING PHENOLIC COMPOUNDS IN SALIFIED FORM

(30) Priorité: 22.05.2008 FR 0802784
(43) Date de publication de la demande: 30.03.2011
(73) Titulaire: Rhodia Opérations, 93306 Aubervilliers (FR)
(72) Inventeur: DESOUHANT-MASSACRET, Magali, F-69300 Caluire (FR)
(74) Mandataire: Dutruc-Rosset, Marie-Claude
(86) Numéro de dépôt international: PCT/EP2009/055934
(87) Numéro de publication internationale: WO 2009/141280

(56) Documents cités:
- EP-A- 0 987 245
- DE-A1- 19 900 382
- FR-A- 2 132 364
- GB-A- 655 555

## Description

La présente invention a pour objet un procédé de séparation de composés phénoliques sous forme salifiée, à partir d'un milieu réactionnel les comprenant.

Plus précisément, l'invention se rapporte à la séparation du gaïacol ou du guétol sous forme salifiée, à partir des milieux de synthèse les comprenant.

L'invention vise plus particulièrement la récupération du gaïacol sous forme de sel de sodium présent en quantité excédentaire au cours de la synthèse de la vanilline ou 4-hydroxy-3-méthoxybenzaldéhyde.

Les aldéhydes hydroxy- et alkoxyaromatiques sont des produits très importants utilisés comme arômes et parfums et comme produits intermédiaires dans de nombreux domaines tels que par exemple, l'agrochimie, la pharmacie, la cosmétique et autres industries.

Les ortho- et para-hydroxybenzaldéhydes, le 4-hydroxy-3-méthoxybenzaldéhyde et le 3-éthoxy-4-hydroxybenzaldéhyde dénommés respectivement « vanilline » et « éthylvanilline » sont parmi les produits les plus importants.

Il a été proposé différents procédés pour la synthèse des aldéhydes aromatiques.

Les procédés les plus importants sont basés sur la fonctionnalisation d'un composé phénolique au départ, phénol, dérivé du catéchol, gaïacol (ou 2-méthoxyphénol), guétol (ou 2-éthoxyphénol).

Intervient, généralement, dans ce type de procédé, le composé phénolique sous une forme salifiée, par exemple, sous forme d'un sel de sodium.

Ainsi, par exemple, de nombreux procédés de préparation de la vanilline font intervenir un sel du gaïacol comme substrat sur lequel est ensuite additionné un groupe formyle en position para du groupe hydroxyle, selon différentes méthodes.

Une voie d'accès classique à la vanilline implique une réaction de condensation de l'acide glyoxylique sur le gaïacol, en milieu basique, pour obtenir l'acide 4-hydroxy-3-méthoxymandélique. Ce produit est ensuite oxydé pour conduire à la vanilline.

La réaction est habituellement conduite en présence de soude et avec un excès de gaïacol : l'acide glyoxylique étant le réactif déficitaire.

Ainsi, en fin de réaction de condensation, on obtient un mélange réactionnel aqueux comprenant le sel de sodium de l'acide 4-hydroxy-3-méthoxymandélique, précurseur de la vanilline, des produits secondaires comme les sels de sodium des acide 2-hydroxy-3-méthoxymandélique et acide 4-hydroxy-5-méthoxy-1,3-dimandélique et un excès plus ou moins important de gaïacolate de sodium.

Ainsi, dans ce milieu réactionnel, il y a présence de plusieurs types de composés phénoliques salifiés à savoir le gaïacol en excès sous forme de gaïacolate de sodium et les produits de la réaction qui sont également des composés phénoliques salifiés tels que les sels de sodium des acides 4-hydroxy-3-méthoxymandélique, 2-hydroxy-3-méthoxymandélique et 4-hydroxy-5-méthoxy-1,3-dimandélique

Pour des considérations économiques, il importe de récupérer le substrat de départ qui n'a pas réagi. Toutefois, l'opération n'est pas aisée car le gaïacol est sous forme de gaïacolate de sodium et est en présence de composés phénoliques également salifiés et de structure proche. Dans EP 0 987245 le phénol est séparé des acides mandéliques salifiés par fixation sur une résine échangeuse d'anions.

Dans certains procédés décrits dans l'état de la technique, en particulier dans FR 2 132 364, le gaïacolate de sodium en fin de réaction de condensation est transformé en gaïacol par un traitement acide, le plus souvent l'acide sulfurique.

On extrait alors le gaïacol non converti de la solution acide par un traitement d'extraction à l'aide d'un hydrocarbure, par exemple le benzène ou le toluène.

L'inconvénient d'un tel procédé est de faire appel à un solvant organique ce qui induit des opérations supplémentaires de distillation afin de pouvoir recycler le solvant organique et le substrat récupéré. De plus, au cours de la distillation, il y a des réactions secondaires conduisant à la formation de lourds.

Par ailleurs, la neutralisation du gaïacolate de sodium par l'acide sulfurique génère du sulfate de sodium ce qui entraîne la formation d'effluents salins importants.

De plus, le gaïacol récupéré doit être à nouveau salifié pour être introduit dans la réaction de condensation avec l'acide glyoxylique.

De même, le milieu réactionnel comprenant les composés mandéliques avec un groupe hydroxyle libre, doit être à nouveau salifié pour être introduit dans la réaction d'oxydation permettant d'obtenir la vanilline.

Pour pallier ces inconvénients, l'invention propose un procédé permettant de récupérer l'excès de composé phénolique de départ sous forme salifiée, en particulier le gaïacolate de sodium selon un procédé qui ne fait pas intervenir cette étape de neutralisation du gaïacolate de sodium en gaïacol, ledit gaïacol devant être extrait à l'aide d'un solvant organique qu'il faut ensuite séparer par une distillation.

Il a maintenant été trouvé et c'est ce qui constitue l'objet de la présente invention, un procédé de séparation de composés phénoliques sous forme salifiée à partir d'un milieu réactionnel aqueux résultant de la réaction d'un composé phénolique et de l'acide glyoxylique en présence d'une base conduisant à un milieu réactionnel comprenant au moins l'excès de composé phénolique de départ sous forme salifiée et les différents composés mandéliques sous forme salifiée résultant de la réaction, caractérisé par le fait qu'on met en contact ledit milieu réactionnel avec une résine échangeuse d'anions basique conduisant à la fixation sélective du composé phénolique de départ sur ladite résine et à la récupération d'un flux aqueux comprenant les composés mandéliques sous forme salifiée issus de la réaction et que l'on sépare le composé phénolique sous forme salifiée fixé sur la résine par un traitement de régénération de la résine.

Dans l'exposé qui suit de la présente invention, on entend "par composé phénolique de départ", un composé benzénique dont au moins un atome d'hydrogène directement lié au noyau benzénique est substitué par un groupe hydroxyle.

Conformément au procédé de l'invention, il a été trouvé dans le cas du traitement d'un milieu réactionnel aqueux comprenant du gaïacolate de sodium, que celui-ci pouvait se fixer sur la résine sous une forme salifiée ce qui permettait ensuite de le recycler à l'étape de synthèse sans être obligé de passer par une étape d'acidification du gaïacolate de sodium pour le transformer en gaïacol qui est récupéré puis ensuite soumis à un nouveau traitement basique car c'est un phénolate qui est mis en oeuvre à l'étape de condensation.

Afin d'illustrer le procédé de l'invention, la Demanderesse cite le cas de la séparation du gaïacolate de sodium à partir du milieu aqueux de condensation du gaïacolate de sodium et de l'acide glyoxylique.

Toutefois, le procédé de l'invention n'est pas limité à la séparation de ce substrat et convient également pour des composés phénoliques de départ sous forme salifiée répondant à la formule suivante : 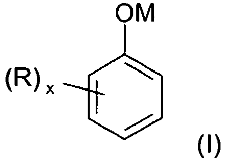 dans ladite formule :
- R est un groupe alkyle ou alkoxy ayant de 1 à 4 atomes de carbone, un atome d'halogène,
- x est un nombre allant de 0 à 3, et plus préférentiellement égal à 1,
- M représente un cation d'un élément métallique du groupe (IA) de la classification périodique à savoir le lithium, sodium, potassium, rubidium et césium ou un cation ammonium.

Dans la formule (I), M est de préférence le sodium.

Comme exemples de groupes alkyle, on peut citer les groupes alkyle, linéaire ou ramifié ayant de 1 à 4 atomes de carbone, tels que méthyle, éthyle, n-propyle, isopropyle, n-butyle, isobutyle, sec-butyle, tert-butyle. Parmi ceux-ci, les groupes méthyle et éthyle sont préférés.

Comme exemples de groupes alkoxy linéaires ou ramifiés ayant de 1 à 4 atomes de carbone, on peut mentionner les groupes méthoxy, éthoxy, n-propoxy, isopropoxy, n-butoxy, isobutoxy, sec-butoxy. Les groupes méthoxy et éthoxy sont préférés.

R représente également un atome d'halogène, de préférence fluor, chlore et brome et plus préférentiellement fluor.

Pour ce qui est de la nature du R, il est à noter que la liste des substituants donnée n'est pas limitative et d'autres substituants peuvent être envisagés dans la mesure où ceux-ci ne gênent pas la séparation du composé de formule (I).

A titre illustratif de composés répondant à la formule (I), on peut mentionner plus particulièrement les sels des composés choisis dans le groupe constitué par : le phénol, le gaïacol, le 3-méthoxyphénol, le guétol, le 3-éthoxyphénol, le 2-isopropoxyphénol, le 3-isopropoxyphénol, le 2-méthoxy-5-méthylphénol, le 2-méthoxy-6-méthylphénol, le 2-méthoxy-6-tert-butylphénol, le 3-chloro-5-méthoxyphénol, le 2,3-diméthoxy-5-méthylphénol, le 2,3-diméthoxyphénol, le 2,6-diméthoxyphénol, le 3,5-diméthoxyphénol, les crésols, le tert-butylphénol, le 2-méthoxyphénol, le 4-méthoxyphénol.

Les composés de formule (I) préférés sont le phénol, le gaïacol, le guétol.

Selon l'invention, on applique le procédé de l'invention au milieu réactionnel aqueux issu de la réaction d'un composé phénolique sous forme salifiée de formule (I) et de l'acide glyoxylique.

La réaction de condensation du composé phénolique de formule (I) et de l'acide glyoxylique peut être conduite en présence d'un hydroxyde d'ammonium mais plus préférentiellement en présence d'un hydroxyde de métal alcalin qui peut être l'hydroxyde de sodium ou de potassium. Pour des considérations économiques, on choisit de préférence l'hydroxyde de sodium.

Pour ce qui est de l'acide glyoxylique, on fait appel à une solution aqueuse d'acide glyoxylique ayant une concentration variant par exemple, entre 15 et 70 % en poids.

On fait réagir l'acide glyoxylique sur le composé phénolique de formule (I) en excès. Le rapport molaire entre le composé phénolique de formule (I) et l'acide glyoxylique varie entre 1,1 et 4,0, de préférence entre 1,5 et 3,0.

La solution d'hydroxyde de métal alcalin mise en oeuvre a une concentration généralement comprise entre 10 et 50 % en poids.

La quantité d'hydroxyde de métal alcalin introduite dans le milieu réactionnel tient compte de la quantité nécessaire pour salifier la fonction hydroxyle du composé phénolique de formule (I) et de la quantité nécessaire pour salifier la fonction carboxylique de l'acide glyoxylique.

La concentration du composé phénolique de formule (I) est comprise de préférence entre 0,5 et 1,5 moles/litre.

La température de la réaction est choisie avantageusement entre 20°C et 60°C.

La réaction est conduite à pression atmosphérique mais sous atmosphère contrôlée de gaz inertes, de préférence d'azote ou de gaz rares, en particulier l'argon. On choisit préférentiellement l'azote.

Après mise en contact du composé phénolique de formule (I), de l'acide glyoxylique et de l'hydroxyde de métal alcalin, on maintient le milieu réactionnel sous agitation et à la température choisie dans l'intervalle précité pendant une durée variable allant de 1 à 10 heures.

En fin de réaction, on obtient un milieu réactionnel aqueux comprenant l'excès de composé phénolique sous forme salifiée répondant à la formule (I) et différents composés mandéliques sous forme salifiée désignés par l'expression « composés mandéliques » et répondant aux formules suivantes : 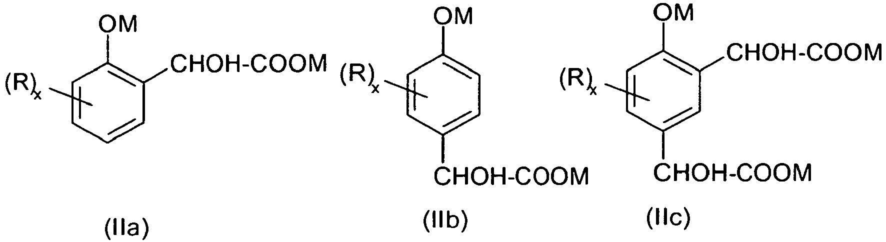 dans lesdites formules, M, R et x ont la signification donnée pour la formule (I).

Les composés mandéliques préférés répondent aux formules (IIa), (IIb) et (IIc) dans lesquelles M représente un atome de sodium, x est un nombre allant de 0 à 3, de préférence égal à 1 et les groupes R, identiques ou différents, représentent un groupe alkyle ou alkoxy ayant de 1 à 4 atomes de carbone, de préférence un groupe méthoxy ou éthoxy ou un atome d'halogène.

L'invention s'applique plus particulièrement dans le cadre de la préparation de la vanilline à un milieu aqueux comprenant du gaïacolate de sodium et des composés mandéliques sous forme salifiée : acides 4-hydroxy-3-méthoxymandélique, 2-hydroxy-3-méthoxymandélique, 4-hydroxy-5-méthoxy-1,3-dimandélique sous forme salifiée.

L'invention s'applique également préférentiellement à la préparation de l'éthylvanilline à un milieu réactionnel qui est un milieu aqueux comprenant du guétolate de sodium et des composés mandéliques sous forme salifiée : acides 3-éthoxy-4-hydroxymandélique, 3-éthoxy-2-hydroxymandélique, 5-éthoxy-4-hydroxy-1,3-dimandélique sous forme salifiée.

La concentration pondérale du composé phénolique de départ sous forme salifiée, de préférence le gaïacolate ou le guétolate de sodium varie généralement entre 1 % et 20 % en poids, de préférence entre 5 % et 10 % en poids.

La concentration pondérale de composés mandéliques (o- p- et dimandélate) dans le milieu réactionnel est habituellement comprise entre 3 % et 30 % en poids, de préférence entre 5 % et 20 % en poids.

Le procédé de l'invention de séparation du composé phénolique sous forme salifiée, est donc mis en oeuvre sur le milieu réactionnel aqueux tel que précédemment défini.

Dans le cas de la préparation de la vanilline et de l'éthylvanilline, il comprend donc du gaïacolate de sodium ou du guétolate de sodium et différents produits de réaction à savoir des mandélates de sodium, mono- ou difonctionnels tels que précisés selon les formules (IIa), (IIb) et (IIc) avec prépondérance du composé de formule (IIb).

Conformément au procédé de l'invention, on met en contact le milieu réactionnel tel que précédemment décrit avec une résine basique conduisant à la fixation sur la résine du composé phénolique sous forme salifiée et à la récupération d'un flux aqueux comprenant les produits de la réaction.

On met donc en contact le milieu réactionnel à traiter avec une résine échangeuse d'anions basique faiblement basiques.

Les résines sont des structures polymériques qui portent des groupes fonctionnels tels que des groupes ammonium quaternaire ou amino primaire, secondaire ou tertiaire.

Ainsi, les résines peuvent comprendre un ou plusieurs groupes fonctionnels répondant aux formules suivantes : 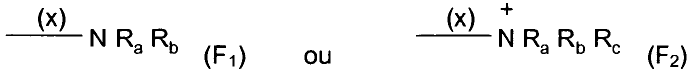 dans lesdites formules :
- les groupes Rₐ. R_{b}, R_{c}, identiques ou différents, représentent un groupe alkyle C₁-C₄, de préférence méthyle ou éthyle, un groupe phényle, un groupe benzyle, un groupe hydroxyalkyle, de préférence un groupe β-hydroxyéthyle,
- au plus deux des groupes Rₐ, R_{b}, R_{c}, sont des atomes d'hydrogène,
- (x) symbolise la liaison reliant l'atome d'azote à la structure polymérique.

Un groupe privilégié de résines convenant bien au procédé de l'invention est constitué par des résines formées d'un squelette polystyrénique qui porte des groupes fonctionnels basiques tels que précédemment définis.

Le squelette polystyrénique est obtenu par polymérisation du styrène et du divinylbenzène, sous l'influence d'un catalyseur d'activation, le plus souvent un peroxyde organique, ce qui conduit à un polystyrène réticulé. La polymérisation se fait le plus souvent en suspension et l'on obtient des billes ou des granules de polymère. Généralement ces billes de polystyrène réticulé sont fonctionnalisées en les traitant avec du chlorométhyléther, en milieu anhydre et en présence d'un catalyseur (par exemple AlCl₃ ou SnCl₄) puis le polystyrène chlorométhylé obtenu désigné par polymère fonctionnalisé P est mis à réagir avec une amine ou de l'ammoniac permettant ainsi de remplacer le chlore du groupe chlorométhylé par le groupe fonctionnel basique.

L'amine est avantageusement une amine secondaire ou tertiaire et répond à la formule suivante :

N RₐR_{b}R_{c} (III)

dans ladite formule :
- les groupes Rₐ, R_{b}, R_{c}, identiques ou différents, représentent un groupe alkyle C₁-C₄, de préférence méthyle ou éthyle, un groupe phényle, un groupe benzyle, un groupe hydroxyalkyle, de préférence un groupe β-hydroxyéthyle,
- au plus deux des groupes Rₐ, R_{b}, R_{c}, sont des atomes d'hydrogène,

Ainsi, dans le procédé de l'invention, on choisit une résine faiblement basique.

Sont considérées comme faiblement basiques, les résines qui portent des fonctions amino secondaire (de type -NHRₐ) ou tertiaire (de type -NRₐR_{b}).

Elles sont obtenues respectivement par réaction du polymère fonctionnalisé P avec une amine primaire Rₐ-NH₂ ou une amine secondaire RₐR_{b}NH. La méthylamine et la diméthylamine sont des exemples de telles amines.

Les résines faiblement basiques les plus courantes sont celles qui portent des fonctions amine tertiaire.

Lorsque le polymère fonctionnalisé P est mis à réagir avec une amine tertiaire, la résine obtenue porte une fonction ammonium quaternaire -N⁺RₐR_{b}R_{c} lui conférant un caractère basique fort.

Les résines ayant un squelette polystyrénique sont mises en oeuvre préférentiellement dans le procédé de l'invention. Toutefois, l'invention n'exclut pas la mise en oeuvre de résines ayant un squelette d'une autre nature dans la mesure où elle porte les groupes basiques adéquates.

Ainsi, conviennent également au procédé de l'invention, les résines polyacryliques.

Leur préparation est analogue à celle des résines polystyréniques.

On prépare des billes à partir d'un ester acrylique et de divinylbenzène copolymérisés en suspension en présence d'un catalyseur d'activation par radical libre. Le polyester acrylique ainsi formé est mis à réagir avec une polyamine contenant au moins une fonction amine primaire et une fonction amine secondaire ou tertiaire. La fonction amine primaire réagit avec la fonction ester du polymère conduisant à une fonction amide tandis que l'autre groupe fonctionnel amino tel que défini constitue le groupe actif échangeur d'anions.

De nombreuses résines sont des produits disponibles sur le marché sous forme sèche ou humide. L'une ou l'autre des formes peuvent être utilisées dans le procédé de l'invention.

Les résines en cause peuvent être du type gel ou du type macroporeux : ce dernier type de résines est préféré.

Elles se présentent habituellement sous forme de particules sensiblement sphériques ayant un diamètre variant de 0,3 à 1,5 mm, de préférence entre 0,3 et 1,2 mm.

La concentration en sites actifs (basiques) de la résine varie avantageusement entre 1 et 3 milliéquivalents de sites actifs par litre de polymère sec, et, de préférence, entre 1,2 et 1,8 milliéquivalents de sites actifs par litre de polymère sec.

Il existe sur le marché des résines commercialisées sous différentes dénominations commerciales. On peut citer, entre autres, les résines suivantes : Amberlite IRA96RF, Amberlite IRA 67, Amberlyst^{®} A21, Amberlyst^{®} A23, Amberlyst^{®} A24; commercialisées par Rohm & Haas ; Lewatit MonoPlus^{®} MP64, Lewatit MP 62, Lewatit VP OC 1072, Lewatit MonoPlus^{®} MP64, Lewatit MP 62 WS, Lewatit S 4268, Lewatit S 4228, Lewatit S 4328, Lewatit S 4428, Lewatit S 4528 vendues par Lanxess ; WBG30, WBG30-B, WBMP, WBACR; commercialisées par ResinTech ; Dowex 22, Dowex 66, Dowex MONOSPHERE 66, Dowex MONOSPHERE 77, Dowex MARATHON WBA, Dowex MARATHON WBA-2, Dowex UPCORE Mono WB-500, Dowex M-43, and XUS 43568.00 vendues par Dow Chemical Co. ; Purolite A100, et A847 de Purolite Co. ; XA 3031, XA 3032, XA 3041, XA 3042, XA 3043, XA 3051, XA 3053, XA 3061, XA 3062, XA 3111, XA 3112, XA 3251 commercialisées par Novasep.

Amberjet 4400 et Amberlyst A26 et Dowex 22 sont des exemples de résines basiques fortes qui peuvent convenir.

Parmi les résines précitées, les résines préférées sont les résines faiblement basiques telles que notamment : Amberlyst A21, Amberlite IRA 67, Lewatit MP 64, Lewatit MP 62 et Purolite A100.

Conformément au procédé de l'invention, on fait passer le milieu réactionnel obtenu en fin de réaction de condensation sur la résine échangeuse d'anions.

Le flux est ainsi à une température voisine de la température de condensation comprise entre 20°C et 60°C.

Généralement, la résine est placée dans un réacteur agité ou bien dans une colonne : le milieu étant introduit généralement de haut en bas.

La quantité de résine mise en oeuvre est au moins égale à la quantité de composé phénolique sous forme salifiée à récupérer.

On récupère en pied de colonne un flux aqueux comprenant tous les composés mandéliques sous forme salifiée alors que le composé phénolique sous forme salifiée, de préférence le gaïacolate ou le guétolate de sodium est fixé sur la résine.

Le flux aqueux comprenant les composés mandéliques sous forme salifiée peut être directement engagé à l'opération d'oxydation permettant d'obtenir l'aldéhyde aromatique correspondant aux composés mandéliques sous forme salifiée.

Dans une étape suivante, on récupère le composé phénolique sous forme salifiée, par régénération de la résine.

On choisit de préférence de régénérer la résine à l'aide d'une base.

Comme bases convenant, on peut citer notamment, la soude, l'ammoniaque, le carbonate de sodium.

A cet effet, on effectue un traitement basique, de préférence à l'aide d'une solution aqueuse basique, ayant une concentration de 2 à 10 % en poids, et plus préférentiellement entre 3 et 8 % en poids. La soude est habituellement utilisée.

La quantité de base mise en oeuvre est au moins égale à la quantité de composé phénolique sous forme salifiée à régénérer.

On obtient alors une solution du composé phénolique qui est salifié et donc directement recyclable à l'étape de condensation.

Comme mentionné précédemment, le procédé de l'invention permet de séparer un flux aqueux comprenant les différents composés mandéliques sous forme salifiée.

Ainsi, le procédé de l'invention permet d'accéder aux aldéhydes hydroxyaromatiques correspondant aux formules (IIa), (IIb) et (IIc) dans lesquelles le groupe glycolique de formule -CHOH-COOH est remplacé par un groupe formyle CHO.

La réaction d'oxydation peut être conduite selon les techniques décrites dans la littérature. Ainsi, on peut utiliser les catalyseurs classiquement utilisés dans les réactions d'oxydation des composés mandéliques, en milieu basique.

L'oxydation est généralement conduite par l'oxygène ou l'air sous pression, en présence d'un catalyseur approprié tel que par exemple, les dérivés du chrome, manganèse, fer, cobalt, nickel, cuivre, zinc, bismuth, aluminium, argent, vanadium ou osmium.

On peut mettre en oeuvre tout particulièrement les oxydes, sulfates, halogénures, acétates desdits éléments métalliques.

On peut également utiliser un catalyseur comprenant au moins deux éléments métalliques. On peut se référer plus particulièrement à WO 2008/148760 qui propose la mise en oeuvre d'un système catalytique comprenant au moins deux éléments métalliques M₁ et M₂ choisis dans le groupe formé par : le cuivre, le nickel, le cobalt, le fer et le manganèse.

Ainsi, l'invention permet d'accéder facilement aux hydroxybenzaldéhydes et plus particulièrement à la vanilline et ses analogues, par exemple 3-éthylvanilline, 3-isopropylvanilline, par oxydation respectivement de l'acide p-hydroxymandélique et des acides 4-hydroxy-3-méthoxymandélique, 3-éthoxy-4-hydroxy-mandélique, ou 4-hydroxy-3-isopropoxymandélique.

On donne ci-après, des exemples de réalisation de l'invention donnés à titre illustratif.

Dans les exemples, la sélectivité de la réaction est définie comme le rapport [gaiacol]fixé/([gaiacol]fixé+[mandélate]fixé).

### Exemple 1

Dans cet exemple, la résine utilisée est une résine Amberlyst A21 qui est une résine échangeuse d'anions faiblement basique fonctionnalisée par des amines tertiaires.

Elle se présente sous forme de billes d'un diamètre moyen compris entre 0,490 et 0,690 mm. On définit le diamètre moyen comme étant tel que 50 % en poids des billes ont un diamètre supérieur ou inférieur au diamètre moyen.

Sa capacité totale d'échange est de 1,3 min.eq/l.

Sa surface spécifique BET est de 35 m²/g, le diamètre moyen des pores de 110Ä et le volume poreux total de 0,10 cm³/g.

Dans un tube shott, on charge 36,70 g de flux comprenant 2,14 g de gaïacolate de sodium (0,014 mol) et 2,54 g de p-mandélate de sodium (0,013 mol) de formule :

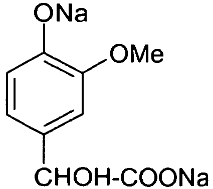

On ajoute ensuite 4,2 g de résine Amberlyst 21 et 10,10 g d'eau.

On suit ensuite l'échange d'anion par analyse chromatographie liquide haute performance de ladite solution.

Dès l'ajout de résine dans le flux, 30 % de gaïacolate sont fixés.

Au bout de 35 min, on fixe 45 % de gaïacolate sans fixer de mandélates. Le rapport qui définit la sélectivité est de 1.

### Exemple 2

Dans cet exemple, on met en oeuvre la résine Lewatit MP64 qui est une résine échangeuse d'anions faiblement basique à base d'un copolymère styrène-divinylbenzène de structure macroporeuse à groupements fonctionnels ammonium tertiaire/quaternaire.

Elle se présente sous forme de billes d'un diamètre uniforme (monodispersé) : 90 % du volume des billes ont un diamètre compris entre 0,54 et 0,64 mm.

Sa capacité totale d'échange est de 1,3 min.eq/I.

On reproduit l'exemple 1 à la seule différence que la nature de la résine est changée.

Avec cette résine, la sélectivité obtenue est de 1.

### Exemple 3

Dans cet exemple, on met en oeuvre la résine Amberlite IRA 67 qui est une résine échangeuse d'anions faiblement basique dont la matrice est de type gel acrylique fonctionnalisée par des amines tertiaires.

Sa capacité totale d'échange est de 1,6 min.eq/l.

On reproduit l'exemple 1 à la seule différence que la nature de la résine est changée

Avec cette résine, la sélectivité obtenue est de 1.

### Exemple 4

Dans cet exemple, on met en oeuvre la résine Purolite A100 qui est une résine échangeuse d'anions faiblement basique dont la matrice est de type polystyrénique fonctionnalisée par des amines tertiaires.

Elle se présente sous forme de billes d'un diamètre moyen compris entre 0,6 et 0,85 mm.

Sa capacité totale d'échange est de 1,3 min.eq/I.

La résine est mise en oeuvre dans une colonne de 300 mL, d'un diamètre de 2,8 cm et d'une hauteur de 24 cm.

Le volume de la résine est de 150 mL.

La colonne est maintenue sous pression atmosphérique à 35°C.

Sur cette résine, on fait percoler à une vitesse de 1,5 m/h, un flux comprenant 5,2 % de gaïacolate de sodium et 5,9 % de composés mandéliques sous forme de sel de sodium (o-, p- et dimandélate) issus d'une réaction de condensation entre l'acide glyoxylique et le gaïacol, en présence de soude conduite selon l'enseignement de l'état de la technique (WO 99/65853) et qui répondent aux formules (IIa), (IIb) et (IIc) dans lesquelles R représente un groupe méthoxy, x est égal à 1 et M est le sodium.

Dans cet exemple, on percole sur la résine 380 g du flux tel que défini précédemment.

On récupère à la sortie de la colonne, 380 g d'un flux exempt de gaïacolate de sodium et contenant la totalité des composés mandéliques sous forme de sels de sodium chargés.

Le rapport qui définit la sélectivité est de 1.

On récupère le gaïacolate de sodium fixé sur la résine par traitement à l'aide de soude.

Une solution aqueuse de soude à 5 % en poids est percolée à travers la résine à une vitesse de 2 m/s.

On recueille un flux contenant le gaïacolate de sodium avec un rendement de 88 % en poids, le rendement étant défini comme le rapport pondéral (en %) entre le gaïacolate engagé et le gaïacolate récupéré.

Celui-ci peut être directement recyclé à l'étape de condensation.

Le flux en sortie de colonne qui contient les composés mandéliques sous forme salifiée est ensuite oxydé sans ajout supplémentaire de solution aqueuse de soude.

Le flux est chargé dans un réacteur en inox 316L équipé d'une agitation mécanique, de contre-pâles et d'une arrivée d'air.

A ce milieu réactionnel est ajouté un système catalytique comprenant C_{O}Cl₂,6H₂O et CuSO₄,5H₂O mis en oeuvre respectivement en une quantité exprimée en pourcentage molaire de composés mandéliques de 0,125 et 0,125.

Le milieu est ensuite chauffé jusqu'à 80°C et l'air est introduit à un débit de 1,6 L/h.

Au bout de 30 minutes de réaction on obtient une sélectivité de la réaction en vanilline de 98 %.

### Exemple 5

On reproduit l'exemple 4 à la différence près que le milieu réactionnel percolé résulte de la réaction du guétol et de l'acide glyoxylique, en présence de soude et comprend donc du guétolate de sodium et les composés mandéliques sous forme de sels de sodium (o-, p- et dimandélate) qui répondent aux formules (IIa), (IIb) et (IIc) dans lesquelles R représente un groupe éthoxy, x est égal à 1 et M est le sodium.

On récupère en sortie de colonne, un flux exempt de guétolate de sodium et contenant la totalité des composés mandéliques sous forme de sels de sodium chargés.

Avec cette résine, le rapport qui définit la sélectivité est de 1.

## Revendications

1. Procédé de séparation de composés phénoliques sous forme salifiée à partir d'un milieu réactionnel aqueux résultant de la réaction d'un composé phénolique et de l'acide glyoxylique en présence d'une base conduisant à un milieu réactionnel comprenant au moins l'excès de composé phénolique de départ sous forme salifiée et les différents composés mandéliques sous forme salifiée résultant de la réaction, **caractérisé par le fait qu'**on met en contact ledit milieu réactionnel avec une résine échangeuse d'anions basique conduisant à la fixation sélective du composé phénolique de départ sur ladite résine et à la récupération d'un flux aqueux comprenant les composés mandéliques sous forme salifiée issus de la réaction et que l'on sépare le composé phénolique sous forme salifiée fixé sur la résine par un traitement de régénération de la résine, ladite résine étant une résine de structure polymérique portant des groupes fonctionnels choisis parmi les groupes ammonium quaternaire ou amino primaire, secondaire ou tertiaire.

2. Procédé selon la revendication 1 **caractérisé par le fait que** le milieu réactionnel de départ comprend l'excès de composé phénolique de départ sous forme salifiée répondant à la formule suivante : 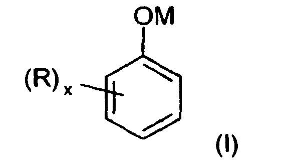 dans ladite formule :
- R est un groupe alkyle ou alkoxy ayant de 1 à 4 atomes de carbone, un atome d'halogène,
- x est un nombre allant de 0 à 3, et plus préférentiellement égal à 1,
- M représente un cation d'un élément métallique du groupe (IA) de la classification périodique à savoir le lithium, sodium, potassium, rubidium et césium ou un cation ammonium.

3. Procédé selon l'une des revendications 1 et 2 **caractérisé par le fait que** le composé phénolique sous forme salifiée de formule (I) est le gaïacolate de sodium ou le guétolate de sodium.

4. Procédé selon l'une des revendications 1 à 3 **caractérisé par le fait que** le milieu réactionnel comprend le composé phénolique sous forme salifiée à une concentration comprise entre 1 et 20 % en poids, de préférence entre 5 et 10 % en poids.

5. Procédé selon l'une des revendications 1 à 4 **caractérisé par le fait que** le milieu réactionnel comprend les produits issus de la réaction du composé phénolique de formule (I) et de l'acide glyoxylique, en présence d'une base.

6. Procédé selon la revendication 5 **caractérisé par le fait que** le milieu réactionnel comprend les composés mandéliques sous forme salifiée répondant aux formules suivantes : 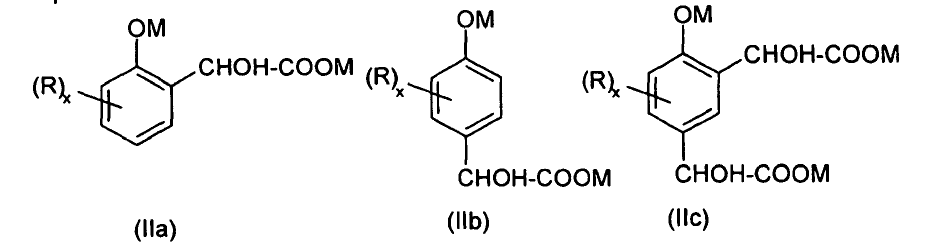 dans lesdites formules, M, R et x ont la signification donnée dans la revendication 2.

7. Procédé selon la revendication 6 **caractérisé par le fait que** les composés mandéliques répondent aux formules (IIa), (IIb) et (IIc) dans lesquelles M représente un atome de sodium, x est un nombre allant de 0 à 3, de préférence égal à 1 et les groupes R, identiques ou différents, représentent un groupe alkyle ou alkoxy ayant de 1 à 4 atomes de carbone, de préférence un groupe méthoxy ou éthoxy ou un atome d'halogène.

8. Procédé selon l'une des revendications 5 à 7 **caractérisé par le fait que** le milieu réactionnel est un milieu aqueux comprenant du gaïacolate de sodium et des composés mandéliques sous forme salifiée : acides 4-hydroxy-3-méthoxymandélique, 2-hydroxy-3-méthoxymandélique, 4-hydroxy-5-méthoxy-1,3-dimandélique sous forme salifiée.

9. Procédé selon l'une des revendications 5 à 7 **caractérisé par le fait que** le milieu réactionnel est un milieu aqueux comprenant du guétolate de sodium et des composés mandéliques sous forme salifiée : acides 3-éthoxy-4-hydroxymandélique, 3-éthoxy-2-hydroxymandélique, 5-éthoxy-4-hydroxy-1,3-dimandélique sous forme salifiée.

10. Procédé selon l'une des revendications 5 à 9 **caractérisé par le fait que** le milieu réactionnel comprend des produits de réaction qui sont des acides mandéliques sous forme salifiée à une concentration comprise entre 3 % et 30 % en poids, de préférence entre 5 % et 20 % en poids.

11. Procédé selon l'une des revendications 1 à 10 **caractérisé par le fait que** la résine comprend un ou plusieurs groupes fonctionnels répondant aux formules suivantes : 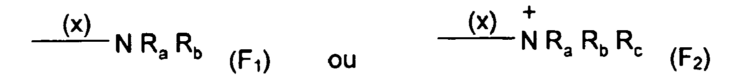 dans lesdites formules :
- les groupes Rₐ, R_{b}, R_{c}, identiques ou différents, représentent un groupe alkyle C₁-C₄, de préférence méthyle ou éthyle, un groupe phényle, un groupe benzyle, un groupe hydroxyalkyle, de préférence un groupe β-hydroxyéthyle,
- au plus deux des groupes Rₐ, R_{b}, R_{c}, sont des atomes d'hydrogène,
- (x) symbolise la liaison reliant l'atome d'azote à la structure polymérique.

12. Procédé selon l'une des revendications 1 à 11 **caractérisé par le fait que** le squelette polymérique est de type polystyrénique ou polyacrylique.

13. Procédé selon l'une des revendications 1 à 12 à **caractérisé par le fait que** la résine se présente sous forme de particules sensiblement sphériques ayant un diamètre variant de 0,3 à 1,5 mm, de préférence entre 0,3 et 1,2 mm.

14. Procédé selon l'une des revendications 1 à 13 à **caractérisé par le fait que** la concentration en sites actifs (basiques) de la résine varie entre 1 et 3 milliéquivalents de sites actifs par litre de polymère sec, et, de préférence, entre 1,2 et 1,8 milliéquivalents de sites actifs par litre de polymère sec.

15. Procédé selon l'une des revendications 1 à 14 **caractérisé par le fait que** la résine est choisie parmi : Amberlyst A21, Amberlite IRA 67, Lewatit MP 64, Lewatit MP 62 et Purolite A100.

16. Procédé selon l'une des revendications 1 à 15 **caractérisé par le fait que** l'on récupère le composé phénolique sous forme salifiée, par régénération de la résine sous forme de traitement basique, de préférence à l'aide d'une solution aqueuse de soude.

17. Procédé selon l'une des revendications 1 à 16 **caractérisé par le fait que** l'on recycle le composé phénolique sous forme salifiée récupéré à l'étape de condensation.

18. Procédé selon l'une des revendications 1 à 17 **caractérisé par le fait que** le flux aqueux comprenant les composés mandéliques sous forme salifiée issus de la séparation peut être introduit à l'opération d'oxydation permettant d'obtenir les aldéhydes aromatiques correspondants.

## Claims

1. Method of separating phenolic compounds in salified form from an aqueous reaction mixture resulting from the reaction of a phenolic compound and glyoxylic acid in the presence of a base, leading to a reaction mixture comprising at least the excess of phenolic starting compound in salified form and the various mandelic compounds in salified form, resulting from the reaction, **characterized in that** said reaction mixture is contacted with a basic anion-exchange resin, leading to the selective attachment of the phenolic starting compound to said resin, and to the recovery of an aqueous stream comprising the mandelic compounds in salified form obtained from the reaction, and **in that** the phenolic compound in salified form that is attached to the resin is separated by a regenerative treatment of the resin, said resin being a resin of polymeric structure carrying functional groups selected from primary, secondary or tertiary amino or quaternary ammonium groups.

2. Method according to Claim 1, **characterized in that** the starting reaction mixture comprises the excess of phenolic starting compound in salified form corresponding to the following formula: 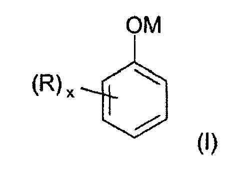 in which formula:
- R is an alkyl or alkoxy group having from 1 to 4 carbon atoms, or a halogen atom,
- x is a number from 0 to 3, and more preferably is 1, and
- M represents a cation of a metallic element from group (IA) of the periodic table, namely lithium, sodium, potassium, rubidium, and cesium, or an ammonium cation.

3. Method according to either of Claims 1 and 2, **characterized in that** the phenolic compound in salified form of formula (I) is sodium guaiacolate or sodium guaetholate.

4. Method according to any of Claims 1 to 3, **characterized in that** the reaction mixture comprises the phenolic compound in salified form at a concentration of between 1% and 20% by weight, preferably between 5% and 10% by weight.

5. Method according to any of Claims 1 to 4, **characterized in that** the reaction mixture comprises the products obtained from the reaction of the phenolic compound of formula (I) and glyoxylic acid, in the presence of a base.

6. Method according to Claim 5, **characterized in that** the reaction mixture comprises the mandelic compounds in salified form corresponding to the following formulae: 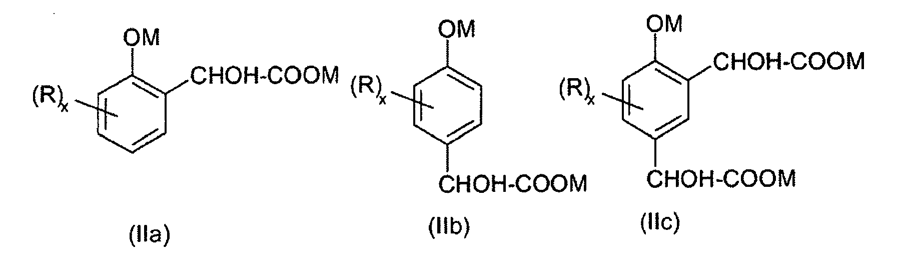 in which formulae M, R, and x are as defined in Claim 2.

7. Method according to Claim 6, **characterized in that** the mandelic compounds correspond to the formulae (IIa), (IIb) and (IIc), in which M represents a sodium atom, x is a number from 0 to 3, and preferably is 1, and the groups R, which are identical or different, represent an alkyl or alkoxy group having from 1 to 4 carbon atoms, preferably a methoxy or ethoxy group, or a halogen atom.

8. Method according to any of Claims 5 to 7, **characterized in that** the reaction mixture is an aqueous mixture comprising sodium guaiacolate and mandelic compounds in salified form: 4-hydroxy-3-methoxymandelic acid, 2-hydroxy-3-methoxymandelic acid, 4-hydroxy-5-methoxy-1,3-dimandelic acid, in salified form.

9. Method according to any of Claims 5 to 7, **characterized in that** the reaction mixture is an aqueous mixture comprising sodium guaetholate and mandelic compounds in salified form: 3-ethoxy-4-hydroxymandelic acid, 3-ethoxy-4-hydroxymandelic acid, 3-ethoxy-2-hydroxymandelic acid, 5-ethoxy-4-hydroxy-1,3-dimandelic acid, in salified form.

10. Method according to any of Claims 5 to 9, **characterized in that** the reaction mixture comprises reaction products which are mandelic acids in salified form at a concentration of between 3% and 30% by weight, preferably between 5% and 20% by weight.

11. Method according to any of Claims 1 to 10, **characterized in that** the resin comprises one or more functional groups corresponding to the following formulae: 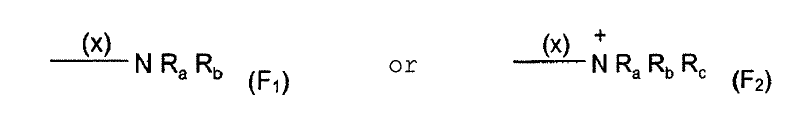 in which formulae:
- the groups Rₐ, R_{b}, and R_{c}, which are identical or different, represent a C₁-C₄-alkyl group, preferably methyl or ethyl, a phenyl group, a benzyl group, or a hydroxyalkyl group, preferably a β-hydroxyethyl group,
- not more than two of the groups Rₐ, R_{b} and R_{c} are hydrogen atoms, and
- (x) symbolizes the bond connecting the nitrogen atom to the polymeric structure.

12. Method according to any of Claims 1 to 11, **characterized in that** the polymeric backbone is a polystyrene or polyacrylic backbone.

13. Method according to any of Claims 1 to 12, **characterized in that** the resin is in the form of substantially spherical particles having a diameter of from 0.3 to 1.5 mm, preferably between 0.3 and 1.2 mm.

14. Method according to any of Claims 1 to 13, **characterized in that** the concentration of (basic) active sites on the resin is between 1 and 3 milliequivalents of active sites per litre of dry polymer, and, preferably, between 1.2 and 1.8 milliequivalents of active sites per litre of dry polymer.

15. Method according to any of Claims 1 to 14, **characterized in that** the resin is selected from the following: Amberlyst A21, Amberlite IRA 67, Lewatit MP 64, Lewatit MP 62 and Purolite A100.

16. Method according to any of Claims 1 to 15, **characterized in that** the phenolic compound in salified form is recovered by regenerating the resin in the form of basic treatment, preferably by means of an aqueous sodium hydroxide solution.

17. Method according to any of Claims 1 to 16, **characterized in that** the phenolic compound in salified form that is recovered is recycled to the condensation step.

18. Method according to any of Claims 1 to 17, **characterized in that** the aqueous stream comprising the mandelic compounds in salified form that are obtained from the separation may be introduced to the oxidizing operation, allowing the corresponding aromatic aldehydes to be obtained.

## Patentansprüche

1. Verfahren zum Abtrennen von Phenolverbindungen in versalzter Form aus einem wässrigen Reaktionsmedium, das aus der Umsetzung einer Phenolverbindung mit Glyoxylsäure in Gegenwart einer Base stammt, die zu einem Reaktionsmedium führt, das mindestens den Überschuss der Ausgangs-Phenolverbindung in versalzter Form und die verschiedenen, sich durch die Umsetzung ergebenden Mandelsäure-Verbindungen in versalzter Form umfasst, **dadurch gekennzeichnet, dass** man das Reaktionsmedium mit einem basischen Anionenaustauscherharz in Kontakt bringt, was zur selektiven Bindung der Ausgangs-Phenolverbindung an das Harz und zur Gewinnung eines wässrigen Stroms führt, der die aus der Umsetzung hervorgegangenen Mandelsäure-Verbindungen in versalzter Form umfasst, und dadurch, dass man die an das Harz gebundene Phenolverbindung in versalzter Form durch eine Regenerationsbehandlung des Harzes abtrennt, wobei es sich bei dem Harz um ein Harz mit polymerer Struktur handelt, das funktionelle Gruppen trägt, die aus quaternären Ammonium- oder primären, sekundären oder tertiären Aminogruppen ausgewählt sind.

2. Verfahren nach Anspruch 1, **dadurch gekennzeichnet, dass** das Ausgangs-Reaktionsmedium den Überschuss an Ausgangs-Phenolverbindung in versalzter Form umfasst, die folgender Formel entspricht: 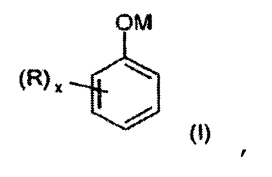 wobei in der Formel:
- R eine Alkyl- oder Alkoxygruppe mit 1 bis 4 Kohlenstoffatomen, ein Halogenatom ist,
- x eine Zahl von 0 bis 3 und besonders bevorzugt gleich 1 ist,
- M für ein Kation eines Metallelements der Gruppe (IA) des Periodensystems, nämlich Lithium, Natrium, Kalium, Rubidium und Cäsium, oder ein Ammoniumkation steht.

3. Verfahren nach einem der Ansprüche 1 und 2, **dadurch gekennzeichnet, dass** die Phenolverbindung in versalzter Form der Formel (I) Natriumguaiacolat oder Natriumguaetholat ist.

4. Verfahren nach einem der Ansprüche 1 bis 3, **dadurch gekennzeichnet, dass** das Reaktionsmedium die Phenolverbindung in versalzter Form in einer Konzentration zwischen 1 Gew.-% und 20 Gew.-%, vorzugsweise zwischen 5 und 10 Gew.-%, umfasst.

5. Verfahren nach einem der Ansprüche 1 bis 4, **dadurch gekennzeichnet, dass** das Reaktionsmedium die Produkte, die aus der Umsetzung der Phenolverbindung der Formel (I) mit Glyoxylsäure in Gegenwart einer Base hervorgehen, umfasst.

6. Verfahren nach Anspruch 5, **dadurch gekennzeichnet, dass** das Reaktionsmedium die Mandelsäure-Verbindungen in versalzter Form der folgenden Formeln umfasst: 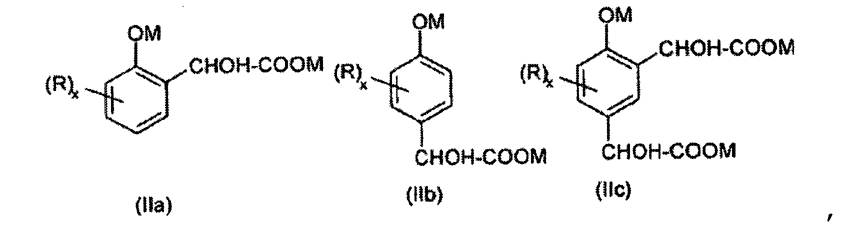 wobei in den Formeln M, R und x die im Anspruch 2 angegebene Bedeutung haben.

7. Verfahren nach Anspruch 6, **dadurch gekennzeichnet, dass** die Mandelsäure-Verbindungen der Formeln (IIa), (IIb) und (IIc), in denen M für ein Natriumatom steht, x eine Zahl von 0 bis 3, vorzugsweise gleich 1, ist und die Gruppen R, die gleich oder verschieden sind, für eine Alkyl- oder Alkoxygruppe mit 1 bis 4 Kohlenstoffatomen, vorzugsweise eine Methoxy- oder Ethoxygruppe, oder ein Halogenatom stehen.

8. Verfahren nach einem der Ansprüche 5 bis 7, **dadurch gekennzeichnet, dass** das Reaktionsmedium ein wässriges Medium ist, das Natriumguaiacolat und Mandelsäure-Verbindungen in versalzter Form umfasst: 4-Hydroxy-3-methoxymandelsäure, 2-Hydroxy-3-methoxymandelsäure, 4-Hydroxy-5-methoxy-1,3-dimandelsäure in versalzter Form.

9. Verfahren nach einem der Ansprüche 5 bis 7, **dadurch gekennzeichnet, dass** das Reaktionsmedium ein wässriges Medium ist, das Natriumguaetholat und Mandelsäure-Verbindungen in versalzter Form umfasst: 3-Ethoxy-4-hydroxymandelsäure, 3-Ethoxy-2-hydroxymandelsäure, 5-Ethoxy-4-hydroxy-1,3-dimandelsäure in versalzter Form.

10. Verfahren nach einem der Ansprüche 5 bis 9, **dadurch gekennzeichnet, dass** das Reaktionsmedium Reaktionsprodukte, die Mandelsäuren in versalzter Form sind, in einer Konzentration zwischen 3 Gew.-% und 30 Gew.-%, vorzugsweise zwischen 5 Gew.-% und 20 Gew.-%, umfasst.

11. Verfahren nach einem der Ansprüche 1 bis 10, **dadurch gekennzeichnet, dass** das Harz eine oder mehrere funktionelle Gruppen der folgenden Formeln umfasst: 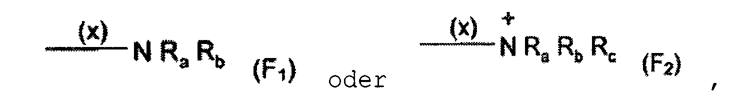 wobei in den Formeln:
- die Gruppen Rₐ, R_{b}, R_{c}, die gleich oder verschieden sind, für eine C₁-C₄-Alkylgruppe, vorzugsweise Methyl- oder Ethylgruppe, eine Phenylgruppe, eine Benzylgruppe, eine Hydroxyalkylgruppe, vorzugsweise eine β-Hydroxyethylgruppe, stehen,
- höchstens zwei der Gruppen Rₐ, R_{b}, R_{c} Wasserstoffatome sind,
- (x) die Bindung symbolisiert, die das Stickstoffatom mit der polymeren Struktur verbindet.

12. Verfahren nach einem der Ansprüche 1 bis 11, **dadurch gekennzeichnet, dass** das Polymer-Grundgerüst vom Polystyrol- oder Polyacrylsäure-Typ ist.

13. Verfahren nach einem der Ansprüche 1 bis 12, **dadurch gekennzeichnet, dass** das Harz in Form von im Wesentlichen kugelförmigen Partikeln mit einem Durchmesser von 0,3 bis 1,5 mm, vorzugsweise zwischen 0,3 und 1,2 mm, vorliegt.

14. Verfahren nach einem der Ansprüche 1 bis 13, **dadurch gekennzeichnet, dass** die Konzentration an aktiven (basischen) Stellen des Harzes zwischen 1 und 3 Milliäquivalenten aktive Stellen pro Liter trockenem Polymer und vorzugsweise zwischen 1,2 und 1,8 Milliäquivalenten aktive Stellen pro Liter trockenem Polymer variiert.

15. Verfahren nach einem der Ansprüche 1 bis 14, **dadurch gekennzeichnet, dass** das Harz aus Amberlyst A21, Amberlite IRA 67, Lewatit MP 64, Lewatit MP 62 und Purolite A100 ausgewählt wird.

16. Verfahren nach einem der Ansprüche 1 bis 15, **dadurch gekennzeichnet, dass** man die Phenolverbindung in versalzter Form durch Regenration des Harzes in Form einer basischen Behandlung, vorzugsweise mithilfe einer wässrigen Natronlauge-Lösung, gewinnt.

17. Verfahren nach einem der Ansprüche 1 bis 16, **dadurch gekennzeichnet, dass** man die Phenolverbindung in versalzter Form, die im Kondensationsschritt gewonnen wird, rückführt.

18. Verfahren nach einem der Ansprüche 1 bis 17, **dadurch gekennzeichnet, dass** der wässrige Strom, der die aus der Abtrennung stammenden Mandelsäure-Verbindungen in versalzter Form umfasst, beim Oxidationsschritt eingebracht werden können, wodurch die entsprechenden aromatischen Aldehyde erhalten werden können.
